# EUROPÄISCHE PATENTSCHRIFT

(11) **EP 4 103 571 B1**
(45) Veröffentlichungstag und Bekanntmachung des Hinweises auf die Patenterteilung: **15.11.2023**
(21) Anmeldenummer: 21702974.3
(22) Anmeldetag: 01.02.2021
(51) Int. Cl.: C07F 9/145, C07C 45/50, C07C 47/02

(54) **NIEDERDRUCKHYDROFORMYLIERUNG VON DIISOBUTEN**
LOW-PRESSURE HYDROFORMYLATION OF DIISOBUTENE
HYDROFORMYLATION BASSE PRESSION DE DIISOBUTÈNE

(30) Priorität: 11.02.2020 EP 20156540
(43) Veröffentlichungstag der Anmeldung: 21.12.2022
(73) Patentinhaber: BASF SE, 67056 Ludwigshafen am Rhein (DE)
(72) Erfinder: SCHWABEN, Jonas, 67056 Ludwigshafen am Rhein (DE); PACIELLO, Rocco, 67056 Ludwigshafen am Rhein (DE); PAPP, Rainer, 67056 Ludwigshafen am Rhein (DE)
(74) Vertreter: BASF IP Association
(86) Internationale Anmeldenummer: PCT/EP2021/052241
(87) Internationale Veröffentlichungsnummer: WO 2021/160448

(56) Entgegenhaltungen:
- EP-A1- 1 099 678

## Beschreibung

C₉-Aldehyde stellen wichtige Zwischenprodukte in der chemischen Industrie dar. Die durch Hydrierung von C₉-Aldehyden erhaltenen C₉-Alkohole finden beispielsweise Verwendung bei der Herstellung von Weichmachern, wohingegen die durch Oxidation von C₉-Alkoholen und/oder C₉-Aldehyden erhaltenen C₉-Carbonsäuren beispielsweise bei der Herstellung von Schmierstoffen, u.a. zur Verwendung in Kälteanlagen, bei der Herstellung von Kosmetika oder bei der Herstellung von Metallsalzen Verwendung finden. Derartige Metallsalze finden beispielsweise Anwendung als Farbentrockner oder PVC-Stabilisatoren.

Industriell werden C₉-Aldehyde durch Hydroformylierung von Cs-Olefinen hergestellt. Bei den zur Hydroformylierung eingesetzten Cs-Olefinen handelt es sich üblicherweise um C₈-Isomerengemische, wie sie durch Dimerisierung von Isobuten erhalten werden. Verfahren zur Dimerisierung von Isobuten sind beispielsweise in der US 2014/0128652 A1, der RU 2270828 C1, der US 2011/0065970 A1, US 5,064,794 A1 oder der EP 1360160 A1 offenbart. Als Hauptprodukte der Dimerisierung von Isobuten werden üblicherweise 2,4,4-Trimethylpent-1-en und 2,4,4-Tri-methylpent-2-en erhalten. Nebenprodukte, die beispielsweise aufgrund von Gerüstisomerisierungsreaktionen während der Dimerisierung von Isobuten entstehen können, sind 2,3,4-Trimethylpentene.

Aufgrund der geringeren Reaktivität von 2,4,4-Trimethylpent-2-en gegenüber 2,4,4-Trimethyl-pent-1-en kommt es bei der Hydroformylierung von Mischungen von 2,4,4-Trimethylpent-1-en und 2,4,4-Trimethylpent-2-en zu einer Aufpegelung von 2,4,4-Trimethylpent-2-en im Reaktionssystem. Hierdurch reduziert sich die Raum-Zeit-Ausbeute und eine Abtrennung des 2,4,4-Tri-methylpent-2-en aus dem Reaktionssystem ist notwendig.

Um das abgetrennte 2,4,4-Trimethylpent-2-en produktiv in der Hydroformylierung zu nutzen, kann dieses beispielsweise zu 2,4,4-Trimethylpent-1-en isomerisiert und anschließend in die Hydroformylierungsreaktion rückgeführt werden. Ein Verfahren zur Isomerisierung von 2,4,4-Trimethylpent-2-en unter Säurekatalyse ist beispielsweise in der US 2,554,251 beschrieben. Dies stellt jedoch einen zusätzlichen Prozessschritt dar und ist daher nicht erstrebenswert.

Um eine Abtrennung von 2,4,4-Trimethylpent-2-en aus dem Reaktionssystem zu vermeiden und 2,4,4-Trimethylpent-2-en zu hydroformylieren, kann die Hydroformylierung bei hohen Drücken mit nicht Liganden-modifizierten Rhodium- oder Cobalt-Komplexen, oder mit Liganden-modifizierten Cobalt-Komplexen mit hoher Isomerisierungswirkung (Shell Prozess) durchgeführt werden. Verfahren zur Hochdruckhydroformylierung und Verfahren zur Hydroformylierung mit Liganden-modifizierten Cobalt-Komplexen sind beispielsweise in Ullmann's Encyclopedia of Industrial Chemistry, Oxo Synthesis, DOI: 10.1002/14356007.a18_312.pub2 oder in J. Falbe, Carbon Monoxide in Organic Synthesis, Springer-Verlag, Berlin Heidelberg, New York, 1970, Seite 70 bis 74 beschrieben. Nachteile an den genannten Verfahren sind die genannten hohen Drücke, und/oder die hohen Hydrierraten der gebildeten Aldehyde zu den entsprechenden Alkoholen. Die dadurch entstehenden komplexen Produktgemische müssen nachgelagert aufwendig getrennt werden.

US 6,403,837 beschreibt ein Verfahren zur Rhodium-katalysierten Hydroformylierung von Di-n-buten bei Drücken, die bevorzugt im Bereich von 10 bis 60 bar liegen unter Verwendung einer Kombination von Organophosphonit- und Organophosphit-Liganden.

In US 4,467,116 wird die Rhodium-katalysierte Hydroformylierung von β-alkylsubstituierten acyclischen α-Olefinen bei Drücken von 2 bis 50 bar unter Verwendung von Organophosphit-Liganden beschrieben.

Ein Verfahren zur Rhodium-katalysierten Hydroformylierung von 2,4,4-Trimethylpent-2-en zu 3,5,5-Trimethylhexanal unter milden Bedingungen und unter Verwendung von Organophosphit-Liganden, das hohe Zeitausbeuten liefert ist im Stand der Technik jedoch nicht bekannt.

Aufgabe der vorliegenden Erfindung war es, ein Rhodium-katalysiertes Verfahren zur Verfügung zu stellen, bei diesem 2,4,4-Trimethylpent-2-en unter milden Bedingungen in hohen Zeitausbeuten zu 3,5,5-Trimethylhexanal in Gegenwart von Organophosphit-Liganden hydroformyliert werden kann. Ebenso soll das Verfahren in der Lage sein, Mischungen von 2,4,4-Trimethyl-pent-1-en und 2,4,4-Trimethylpent-2-en unter milden Bedingungen in hohen Zeitausbeuten zu 3,5,5-Trimethylhexanal zu hydroformylieren. Das erfindungsgemäße Verfahren soll sich zudem durch eine hohe Stabilität der eingesetzten Organophosphit-Liganden auszeichnen. Damit soll sichergestellt werden, dass es nur zu einer geringen Nebenproduktbildung kommt, und im Fall einer kontinuierlichen Verfahrensführung, die Liganden und/oder die Katalysatormetall-Liganden-Komplexe in das Verfahren zurückgeführt werden können. Ebenfalls wird dadurch eine hohe Standzeit des Verfahrens ermöglicht.

Eine hohe Zeitausbeute bzgl. der Hydroformylierung von 2,4,4-Trimethylpent-2-en zu 3,5,5-Trimethylhexanal liegt vor, wenn ein Umsatz des Edukts ≥85% und eine Selektivität von ≥95% innerhalb von 10 h erreicht werden.

Eine hohe Zeitausbeute bzgl. der Hydroformylierung von 2,4,4-Trimethylpent-1-en zu 3,5,5-Trimethylhexanal liegt vor, wenn ein Umsatz des Edukts ≥95% und eine Selektivität von ≥95% innerhalb von 10 h erreicht werden.

Milde Bedingungen liegen vor, wenn der Druck bei dem die Hydroformylierung durchgeführt wird 1 bis 100 bar abs, bevorzugt, 5 bis 80 bar abs, weiter bevorzugt 5 bis 55 bar abs und besonders bevorzugt 8 bis 20 bar abs beträgt und die Temperatur bei der die Hydroformylierung durchgeführt wird 50 bis 200 °C, bevorzugt 80 bis 150 °C, und besonders bevorzugt 90 bis 120°C beträgt.

Bei Nebenprodukten handelt es sich um Abbauprodukte der Liganden und/oder Reaktions- und/oder Folgeprodukte aus der Reaktion zwischen den Abbauprodukten der Liganden und den bei der Hydroformylierung hergestellten Aldehyden.

Die Aufgabe wird gelöst durch ein Hydroformylierungsverfahren zur Herstellung von 3,5,5-Trimethylhexanal umfassend die Umsetzung von 2,4,4-Trimethylpent-2-en mit H₂ und CO in einer Reaktionszone in Gegenwart von einem oder mehreren freien Organophosphit-Liganden der allgemeinen Formel (I) 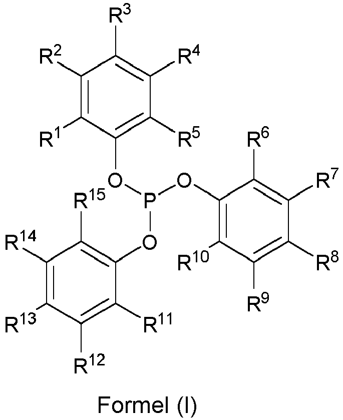 wobei,
R¹, R², R³, R⁴ und R⁵ unabhängig voneinander H, C₁- bis C₉-Alkyl oder C₁- bis C₉-Alkoxyl sind und R¹, R², R³, R⁴ und R⁵ nicht gleichzeitig H sind, und
R⁶, R⁷, R⁸, R⁹ und R¹⁰ unabhängig voneinander H, C₁- bis C₉-Alkyl oder C₁- bis C₉-Alkoxyl sind und R⁶, R⁷, R⁸, R⁹ und R¹⁰ nicht gleichzeitig H sind, und
R¹¹, R¹², R¹³, R¹⁴ und R¹⁵ unabhängig voneinander H, C₁- bis C₉-Alkyl oder C₁- bis C₉-Alkoxyl sind und R¹¹, R¹², R¹³, R¹⁴ und R¹⁵ nicht gleichzeitig H sind,
und eines mit einem oder mehreren Organophosphit-Liganden der allgemeinen Formel (I) komplexierten, homogenen Rhodium-Katalysators bei einem Druck von 1 bis 100 bar abs und einer Temperatur von 50 bis 200°C.

In einer Verbindung der allgemeinen Formel (I) sind:

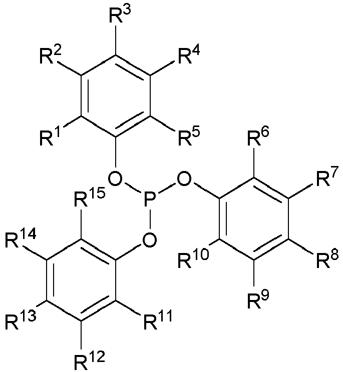

R¹, R², R³, R⁴ und R⁵ sind unabhängig voneinander H, C₁- bis C₉-Alkyl oder C₁- bis C₉-Alkoxyl, wobei R¹, R², R³, R⁴, und R⁵ nicht gleichzeitig H sind. Bevorzugt sind R¹, R², R³, R⁴ und R⁵ unabhängig voneinander H, C₁- bis C₄-Alkyl oder C₁- bis C₄-Alkoxyl, wobei R¹, R², R³, R⁴ und R⁵ nicht gleichzeitig H sind.

R⁶, R⁷, R⁸, R⁹ und R¹⁰ sind unabhängig voneinander H, C₁-bis C₉-Alkyl oder C₁- bis C₉-Alkoxyl, wobei R⁶, R⁷, R⁸, R⁹ und R¹⁰ nicht gleichzeitig H sind. Bevorzugt sind R⁶, R⁷, R⁸, R⁹ und R¹⁰ unabhängig voneinander H, C₁- bis C₄-Alkyl oder C₁- bis C₄-Alkoxyl, wobei R⁶, R⁷, R⁸, R⁹ und R¹⁰ nicht gleichzeitig H sind.

R¹¹, R¹², R¹³, R¹⁴ und R¹⁵ sind unabhängig voneinander H, C₁-bis Cg-Alkyl oder C₁- bis C₉-Alko-xyl, wobei R¹¹, R¹², R¹³, R¹⁴ und R¹⁵ nicht gleichzeitig H sind. Bevorzugt sind R⁶, R⁷, R⁸, R⁹ und R¹⁰ unabhängig voneinander H, C₁- bis C₄-Alkyl oder C₁- bis C₄-Alkoxyl, wobei R¹¹, R¹², R¹³, R¹⁴ und R¹⁵ nicht gleichzeitig H sind.

In einer bevorzugten Ausführungsform der Formel (I) sind:
R¹, R³ und R⁵ unabhängig voneinander H, C₁-bis C₉-Alkyl oder C₁- bis C₉-Alkoxyl, bevorzugt H, C₁- bis C₄-Alkyl oder C₁- bis C₄-Alkoxyl und R² und R⁴ H, wobei R¹, R³ und R⁵ nicht gleichzeitig H sind, und
R⁶, R⁸ und R¹⁰ unabhängig voneinander H, C₁-bis C₉-Alkyl oder C₁- bis C₉-Alkoxyl, bevorzugt H, C₁- bis C₄-Alkyl oder C₁- bis C₄-Alkoxyl und R⁷ und R⁹ H, wobei R⁶, R⁸ und R¹⁰ nicht gleichzeitig H sind, und
R¹¹, R¹³ und R¹⁵ unabhängig voneinander H, C₁-bis C₉-Alkyl oder C₁- bis C₉-Alkoxyl, bevorzugt H, C₁- bis C₄-Alkyl oder C₁- bis C₄-Alkoxyl und R¹² und R¹⁴ H, wobei R¹¹, R¹³ und R¹⁵ nicht gleichzeitig H sind.

In einer weiter bevorzugten Ausführungsform der Formel (I) sind:
R¹ C₁-bis C₉-Alkyl oder C₁- bis C₉-Alkoxyl, bevorzugt C₁- bis C₄-Alkyl oder C₁- bis C₄-Alkoxyl, R³ H, C₁-bis C₉-Alkyl oder C₁- bis C₉-Alkoxyl, bevorzugt H, C₁- bis C₄-Alkyl oder C₁- bis C₄-Alkoxyl und R², R⁴ und R⁵ H, und
R⁶ C₁-bis C₉-Alkyl oder C₁- bis C₉-Alkoxyl, bevorzugt C₁- bis C₄-Alkyl oder C₁- bis C₄-Alkoxyl, R⁸ H, C₁-bis C₉-Alkyl oder C₁- bis C₉-Alkoxyl, bevorzugt H, C₁- bis C₄-Alkyl oder C₁- bis C₄-Alkoxyl und R⁷, R⁹ und R¹⁰ H, und
R¹¹ C₁-bis C₉-Alkyl oder C₁- bis C₉-Alkoxyl, bevorzugt C₁- bis C₄-Alkyl oder C₁- bis C₄-Alkoxyl, R¹³ H, C₁-bis C₉-Alkyl oder C₁- bis C₉-Alkoxyl, bevorzugt H, C₁- bis C₄-Alkyl oder C₁- bis C₄-Al-koxyl und R¹², R¹⁴ und R¹⁵ H.

In einer weiter bevorzugten Ausführungsform der Formel (I) sind:
R¹ und R³ C₁-bis C₉-Alkyl oder C₁- bis C₉-Alkoxyl, bevorzugt C₁- bis C₄-Alkyl oder C₁- bis C₄-Al-koxyl und R², R⁴ und R⁵ H, und
R⁶ und R⁸ C₁-bis C₉-Alkyl oder C₁- bis C₉-Alkoxyl, bevorzugt C₁- bis C₄-Alkyl oder C₁- bis C₄-Al-koxyl und R⁷, R⁹ und R¹⁰ H, und
R¹¹ und R¹³ C₁-bis C₉-Alkyl oder C₁- bis C₉-Alkoxyl, bevorzugt C₁- bis C₄-Alkyl oder C₁- bis C₄-Alkoxyl und R¹², R¹⁴ und R¹⁵ H,
wobei R¹, R³, R⁶, R⁸, R¹¹ und R¹³ gleich sind.

In besonders bevorzugten Ausführungsformen der Formel (I) sind:

| | R¹, R⁶, R¹¹ | R³, R⁸, R¹³ | R², R⁴, R⁵, R⁷, R⁹, R¹⁰, R¹², R¹⁴, R¹⁵ |
|---|---|---|---|
| I) | Methyl | Methyl | H |
| II) | Methyl | H | H |
| III) | Ethyl | Ethyl | H |
| IV) | Ethyl | H | H |
| V) | n-Propyl | n-Propyl | H |
| VI) | n-Propyl | H | H |
| VII) | iso-Propyl | iso-Propyl | H |
| VIII) | iso-Propyl | H | H |
| IX) | n-Butyl | n-Butyl | H |
| X) | n-Butyl | H | H |
| XI) | sec-Butyl | sec-Butyl | H |
| XII) | sec-Butyl | H | H |
| XIII) | iso-Butyl | iso-Butyl | H |
| XIV) | iso-Butyl | H | H |
| XV) | tert-Butyl | tert-Butyl | H |
| XVI) | tert-Butyl | H | H |
| XVII) | Methoxy | Methoxy | H |
| XVIII) | Methoxy | H | H |
| XIX) | Ethoxy | Ethoxy | H |
| XX) | n-Propoxy | n-Propoxy | H |
| XXI) | n-Propoxy | H | H |
| XXII) | iso-Propoxy | iso-Propoxy | H |
| XXIII) | iso-Propoxy | H | H |
| XXIV) | n-Butoxy | n-Butoxy | H |
| XXV) | n-Butoxy | H | H |
| XXVI) | sec-Butoxy | sec-Butoxy | H |
| XXVII) | sec-Butoxy | H | H |
| XXVIII) | iso-Butoxy | iso-Butoxy | H |
| XXIX) | iso-Butoxy | H | H |
| XXX) | tert-Butoxy | tert-Butoxy | H |
| XXXI) | tert-Butoxy | H | H |
| XXXII) | n-Nonyl | H | H |

Insbesonders sind die Ausführungsformen XV) und XVI) bevorzugt.

C₁- bis C₉-Alkyl umfasst gerade oder verzweigte C₁- bis C₉-Alkylgruppen oder cyclische C₃- bis C₉-Alkylgruppen. Bei C₁- bis C₉-Alkyl handelt es sich beispielsweise um Methyl, Ethyl, n-Propyl, iso-Propyl, Cyclopropyl, n-Butyl, sec-Butyl, iso-Butyl, tert-Butyl, n-Pentyl, 2-Methylbutyl, 3-Methylbutyl, Cyclopentyl, n-Hexyl, Cyclohexyl, 2-Methylpentyl, 3-Methylpentyl, 4-Methylpentyl, 3,3-Dimethylbutyl, 2-Ethylbutyl, n-Heptyl, 2-Methylhexyl, 2-Ethylpentyl, 4-Methyl-Cyclohexyl, n-Octyl, iso-Octyl, 2-Ethylhexyl, n-Nonyl oder iso-Nonyl.

C₁- bis C₄-Alkyl umfasst gerade oder verzweigte C₁- bis C₄-Alkylgruppen oder cyclische C₃-Alkylgruppen. Bei C₁- bis C₄-Alkyl handelt es sich beispielsweise um Methyl, Ethyl, n-Propyl, iso-Propyl, Cyclopropyl, n-Butyl, sec-Butyl, iso-Butyl oder tert-Butyl.

C₁- bis C₉-Alkoxyl umfasst gerade oder verzweigte C₁- bis C₉-Alkoxylgruppen oder cyclische C₃-bis C₈-Alkoxylgruppen. Bei C₁- bis C₉-Alkoxyl handelt es sich beispielsweise um Methoxy, Ethoxy, n-Propoxy, iso-Propoxy, Cyclopropoxy, n-Butoxy, sec-Butoxy, iso-Butoxy, tert-Butoxy, n-Pentoxy, 2-Methylbutoxy, 3-Methylbutoxy, Cyclopentoxy, n-Hexoxy, Cyclohexoxy, 2-Methylpentoxy, 3-Methylpentoxy, 4-Methylpentoxy, 3,3-Dimethylbutoxy, 2-Ethylbutoxy, n-Heptoxy, 2-Methylhexoxy, 2-Ethylpentoxy, 4-Methyl-Cyclohexoxy, n-Octoxy, iso-Octoxy, 2-Ethylhexoxy, n-Nonoxy oder iso-Nonoxy.

C₁- bis C₄-Alkoxyl umfasst gerade oder verzweigt C₁- bis C₄-Alkoxygruppen oder cyclische C₃-Alkoxygruppen. C₁- bis C₄-Alkoxyl handelt es sich beispielsweise um Methoxy, Ethoxy, n-Propoxy, iso-Propoxy, Cyclopropoxy, n-Butoxy, sec-Butoxy, iso-Butoxy oder tert-Butoxy.

Neben 2,4,4-Trimethylpent-2-en können auch Mischungen von 2,4,4-Trimethylpent-1-en und 2,4,4-Trimethylpent-2-en in dem erfindungsgemäßen Verfahren eingesetzt werden. Werden Mischungen von 2,4,4-Trimethylpent-1-en und 2,4,4-Trimethylpent-2-en in dem erfindungsgemäßen Verfahren eingesetzt werden beide Isomere mit hohen Zeitausbeuten zu 3,5,5-Trimethylhexanal hydroformyliert.

Mischungen von 2,4,4-Trimethylpent-1-en und 2,4,4-Trimethylpent-2-en werden bevorzugt dann eingesetzt, wenn das zu hydroformylierende 2,4,4-Trimethylpent-2-en aus der Dimerisierung von Isobuten stammt und dadurch gemeinsam mit 2,4,4-Trimethylpent-1-en anfällt.

Aufgrund der unterschiedlichen Dimerisierungsverfahren für Isobuten und/oder der unterschiedlichen Aufreinigungsverfahren des aus der Dimerisierung erhaltenen Diisobutens, kann die Zusammensetzung der Mischungen je nach Herstellungs- und/oder Aufreinigungsverfahren über weite Bereiche variieren.

Mischungen von 2,4,4-Trimethylpent-1-en und 2,4,4-Trimethylpent-2-en weisen bevorzugt ein Mengenverhältnis von 2,4,4-Trimethylpent-1-en zu 2,4,4-Trimethylpent-2-en im Bereich von 99 : 1 bis 1 : 99, besonderes bevorzugt im Bereich von 95 : 5 bis 5 : 95, weiter bevorzugt im Bereich von 80 : 20 bis 20 : 80 und ins besonders bevorzugt im Bereich von 80 : 20 bis 70 : 30 auf, so beispielsweise ein Verhältnis von 75 : 25.

Das Molverhältnis zwischen der Gesamtmenge an Olefin und der Gesamtmenge an Rhodium in der Reaktionszone beträgt bevorzugt 10000 : 1 bis 10 : 1 und weiter bevorzugt 5000 : 1 bis 100 : 1.

Die Gesamtmenge an 2,4,4-Trimethylpent-1-en und 2,4,4-Trimethylpent-2-en an der Gesamtmenge an Olefin im Feed zur Reaktionszone beträgt bevorzugt 10 bis 100 %, besonders bevorzugt 50 bis 100 % und ins besonders bevorzugt 90 bis 100 %. Neben 2,4,4-Trimethylpent-1-en und 2,4,4-Trimethylpent-2-en können noch andere Olefine im Feed zur Reaktionszone vorliegen und ggf. hydroformyliert werden. Solche Olefine sind beispielsweise Butene, wie n-Buten, 2-Buten, Isobuten und/oder Oligomere, wie sie bei der Isobutendimerisierung entstehen können, beispielsweise 2,3,4-Trimethylpentene.

Die Hydroformylierung erfolgt in einer Reaktionszone die einen oder mehrere, gleiche oder verschiedene Reaktoren umfasst. Im einfachsten Fall wird die Reaktionszone durch einen einzelnen Reaktor gebildet. Bei mehreren Reaktoren können die Reaktoren gleiche oder unterschiedliche Vermischungscharakteristiken aufweisen. Die verwendeten Reaktoren können durch Einbauten ein- oder mehrfach unterteilt sein. Wird eine Reaktionszone durch mehr als einen Reaktor gebildet, können die Reaktoren untereinander beliebig verschaltet sein. So können die Reaktoren beispielsweise parallel oder in Reihe geschaltet sein. Als Reaktoren eignen sich prinzipiell alle Reaktortypen, die für Hydroformylierungsreaktionen geeignet sind, wie Rührreaktoren, Blasensäulenreaktoren wie bspw. in US 4778929 A beschrieben, Umlaufreaktoren wie bspw. in EP 11140017 A1 beschrieben, Rohrreaktoren, wobei die einzelnen Reaktoren einer Reihe unterschiedliche Mischungscharakteristiken haben können, wie bspw. in EP 423769 A1 beschrieben oder kompartimentierte Reaktoren wie bspw. in EP 1231198 A1 oder US 5728893 A beschrieben.

Bei dem erfindungsgemäßen Verfahren beträgt die Temperatur in der Reaktionszone 50 bis 200°C. Bevorzugt beträgt die Temperatur in der Reaktionszone bei dem erfindungsgemäßen Verfahren 80 bis 150°C uns besonders bevorzugt 90 bis 120°C.

Bei dem erfindungsgemäßen Verfahren beträgt der Druck in der Reaktionszone 1 bis 100 bar abs. Bevorzugt beträgt der Druck in der Reaktionszone bei dem erfindungsgemäßen Verfahren 5 bis 80 bar abs, weiter bevorzugt 5 bis 55 bar abs und besonders bevorzugt 8 bis 20 bar abs.

Der homogen im Reaktionsmedium gelöste Rhodium-Katalysator wird bevorzugt in situ in der Reaktionszone durch Umsetzung eines Rhodium-Präkursors mit einem oder mehreren Organophosphit-Liganden der allgemeinen Formel (I), CO und H₂ gebildet.

Ebenfalls bevorzugt, kann der Rhodiumkatalysator auch präformiert eingesetzt werden. Hierzu wird der Rhodiumkatalysator durch Umsetzung eines Rhodium-Präkursors mit einem oder mehreren Organophosphit-Liganden der allgemeinen Formel (I), CO und H₂ gebildet. Die Präformierung des Rhodiumkatalysators erfolgt bevorzugt bei einer Temperatur von 50 bis 200 °C, weiter bevorzugt bei einer Temperatur von 80 bis 150 °C und besonders bevorzugt bei einer Temperatur von 90 bis 120 °C. Der Druck bei der Präformierung beträgt bevorzugt 1 bis 100 bar abs, weiter bevorzugt 5 bis 80 bar abs, weiter bevorzugt 5 bis 55 bar abs und besonders bevorzugt 8 bis 20 bar abs. In besonders bevorzugten Fällen wird die Präformierung bei vergleichbarer Temperatur und bei vergleichbarem Druck, wie die Hydroformylierungsreaktion selbst durchgeführt. Der Rhodium-Präkursor ist bevorzugt in einem Lösungsmittel gelöst. Geeignete Lösungsmittel sind beispielsweise Aromaten, wie Toluol oder Xylole, längerkettige Kohlenwasserstoffe, die unter den Reaktionsbedingungen flüssig sind, Ester aliphatischer Carbonsäuren mit Alkanolen, wie Texanol^{®}, Ester aromatischer Carbonsäuren, wie C₈- bis C₁₃-Dialkylterephthalate oder Mischungen aus zwei oder mehr der zuvor genannten Lösungsmittel. Die Präformierung kann in der späteren Reaktionszone oder in einem separaten Reaktor erfolgen. Erfolgt die Präformierung in der späteren Reaktionszone werden die zu hydroformylierenden Olefine der Reaktionszone zugeführt, wenn die Präformierung des Rhodiumkatalysators zumindest weitgehend erfolgt ist.

Bevorzugte Rhodium-Präkursoren sind Rhodiumcarbonyle, Rhodium(I)-Salze, Rhodium(II)-Salze, Rhodium(III)-Salze oder Mischungen aus zwei oder mehr der zuvor genannten Präkursoren. Bevorzugte Rhodiumcarbonyle sind Rh₄(CO)₁₂, Rh₆(CO)₁₆ oder Mischungen davon. Bevorzugte Rhodium(I)-Salze sind Rh(CO)₂acac, Rh₂(CO)₄Cl₂ oder Mischungen davon. Ein bevorzugtes Rhodium(II)-Salz ist Rh₂(OAc)₄. Ein bevorzugtes Rhodium(III)-Salz ist Rh(NO₃)₃.

Rhodium-Präkursor und Organophosphit-Ligand der allgemeinen Formel (I) können gemeinsam oder separat der Reaktionszone zugeführt werden. Rhodium-Präkursor und/oder Organophosphit-Ligand der allgemeinen Formel (I) können dabei direkt oder in einem Lösungsmittel gelöst der Reaktionszone zugeführt werden. Bei dem Lösungsmittel handelt es sich beispielsweise um 3,5,5-Trimethylhexanal oder höhersiedende Reaktionskomponenten, z.B. die Produkte aus der Aldolkondensation von 3,5,5-Trimethylhexanal, Aromaten, wie Toluol oder Xylole, längerkettige Kohlenwasserstoffe die unter den Reaktionsbedingungen flüssig sind, Ester aliphatischer Carbonsäuren mit Alkanolen, wie Texanol^{®}, Ester aromatischer Carbonsäuren, wie C₈- bis C₁₃-Dia-lkylterephthalate oder um Mischungen aus zwei oder mehr der zuvor genannten Lösungsmittel.

Die Rhodiumkonzentration in der Reaktionszone liegt bevorzugt im Bereich von 20 bis 250 Gew. ppm und besonders bevorzugt im Bereich von 60 bis 200 Gew. ppm bezogen auf das Gesamtgewicht der flüssigen Phase in der Reaktionszone.

Methoden zur Ergänzung von Rhodium in der Reaktionszone um eine Abnahme bei der Katalysatoraktivität auszugleichen sind beispielsweise in der US 6700021 A oder in der EP 2836474 A1 beschrieben, wobei das in der EP 2836474 A1 beschriebene Verfahren bevorzugt ist.

Das Molverhältnis der Gesamtmenge an Organophosphit-Liganden der allgemeinen Formel (I) in der Reaktionszone zur Gesamtmenge an Rhodium in der Reaktionszone liegt bevorzugt im Bereich von 1 : 1 bis 100 : 1 und besonders bevorzugt im Bereich von 2 : 1 zu 50 : 1, beispielsweise bei 20 : 1 oder bei 10 : 1.

Freie Organophosphit-Liganden der allgemeinen Formel (I) sind homogen im Reaktionsmedium in der Reaktionszone verteilt. Freie Organophosphit-Liganden der allgemeinen Formel (I) sind nicht mit Rhodium, bzw. anderen Metallen komplexiert.

Das Molverhältnis von H₂ zu CO die der Reaktionszone zugeführt werden beträgt bevorzugt 2 : 1 bis 1 : 2 und bevorzugt 60 : 40 bis 50 : 50.

H₂ und CO werden üblicherweise als Gasgemisch der Reaktionszone zugeführt. Neben H₂ und CO kann das Gasgemisch noch andere Verbindungen wie CH₄, N₂, CO₂ und/oder H₂S enthalten. Der Gehalt an von H₂ und CO unterschiedlichen Verbindungen im Gasgemisch beträgt bevorzugt weniger als 5 vol.%.

Das erfindungsgemäße Verfahren wird bevorzugt in einem Lösungsmittel durchgeführt. Die Verwendung eines Lösungsmittels hat den Vorteil, dass die bei der Hydroformylierung entstehende Reaktionswärme besser verteilt und dadurch "Hot-Spots" in der Reaktionszone vermieden werden. Dadurch können Nebenreaktionen, wie die Degradation des Katalysators oder Kondensationsreaktionen der Aldehyde reduziert werden. Geeignete Lösungsmittel sind unter den Reaktionsbedingungen und während der Aufarbeitung der Reaktionsprodukte inert, d.h. sie gehen keine unerwünschten Reaktionen mit den Edukten, Reaktionsprodukten, Organophosphit-Liganden der allgemeinen Formel (I), Rhodium-Präkursoren und/oder dem Katalysator, dem Katalysatormetall bzw. Katalysatormetall-Liganden-Komplexe ein. Geeignete Lösungsmittel sind beispielsweise 3,5,5-Trimethylhexanal oder höhersiedende Reaktionskomponenten, z.B. die Produkte aus der Aldolkondensation von 3,5,5-Trimethylhexanal. Weiterhin eignen sich Aromaten, wie Toluol oder Xylole, längerkettige Kohlenwasserstoffe, die unter den Reaktionsbedingungen flüssig sind, Ester aliphatischer Carbonsäuren mit Alkanolen, wie Texanol^{®}, Ester aromatischer Carbonsäuren, wie C₈- bis C₁₃-Dialkylterephthalate oder Mischungen aus zwei oder mehr der zuvor genannten Lösungsmittel.

Das erfindungsgemäße Verfahren kann kontinuierlich, semi-kontinuierlich oder diskontinuierlich durchgeführt werden. Eine kontinuierliche oder semi-kontinuierliche Verfahrensführung ist bevorzugt.

Die Abtrennung der Hydroformylierungsprodukte vom Austrag aus der Reaktionszone erfolgt bevorzugt über das Flüssigaustragsverfahren. Der im Wesentlichen flüssige Austrag aus der Reaktionszone wird dabei einer Rektifikation zugeführt, wobei die Hydroformylierungsprodukte als Kopfprodukte erhalten werden. Die so erhaltenen Hydroformylierungsprodukte können dann weiteren Aufarbeitungs- und/oder Umarbeitungsschritten bspw. Hydrierung oder Oxidation zugeführt werden. Es ist bevorzugt, dass der im Wesentlichen flüssige Austrag aus der Reaktionszone in einer oder mehreren Stufen entspannt wird, wobei der Austrag aus jeder Entspannungsstufe in eine flüssige und eine gasförmige Phase getrennt wird. Dadurch können beispielsweise leichter als die Hydroformylierungsprodukte siedende Nebenprodukte, nicht umgesetztes Olefin, Wasserstoff und/oder Kohlenstoffmonoxid aus dem Austrag der Hydroformylierung entfernt werden. Die gasförmige Phase und die flüssige Phase, die auf der letzten Entspannungsstufe anfallen, werden anschließend einer Rektifikationskolonne im Gegenstrom zugeführt. Die Hydroformylierungsprodukte werden überwiegend in der Kopffraktion erhalten. Die hochsiedenden Nebenprodukte werden zusammen mit den Katalysatormetall-Liganden Komplexen, und ggf. deren Abbauprodukte überwiegend in der Sumpffraktion erhalten. Die hochsiedenden Nebenprodukte und die darin gelösten Katalysatormetall-Liganden-Komplexe, und ggf. deren Abbauprodukte können in die Hydroformylierungsreaktion zurückgeführt, oder der Aufarbeitung des Katalysatormetalls zugeführt werden. Vorteilhafte Verfahrensausgestaltungen zum Flüssigaustragsverfahren sind beispielsweise in der EP 0846095 A1 oder der EP 1255720 A1 offenbart. Die in der Kopffraktion anfallenden rohen Hydroformylierungsprodukte können anschließend der weiteren Aufarbeitung zugeführt werden. Ein geeignetes Verfahren zur destillativen Aufreinigung der in der Kopffraktion anfallenden rohen Hydroformylierungsprodukte ist beispielsweise in der DE 19914260 A1 beschrieben. Auch wenn das in der DE 19914260 A1 offenbarte Verfahren auf Alkohole beschränkt ist, liegt es im Rahmen der Fähigkeiten des Fachmanns, das Verfahren auf die destillative Aufreinigung der rohen Hydroformylierungsprodukte anzuwenden.

### Beispiele:

### Beispiel 1:

Unter Argon wurden Rh(CO)₂(acac) (5.1 mg, 0.02 mmol) und Tris(2,4-di-tert-butylphenyl)phosphit (640 mg, 0.99 mmol) in Diisobuten (20.0 g, 178 mmol; 2,4,4-Trimethylpent-1-en / 2,4,4-Tri-methylpent-2-en: 80:20 bis 75:25) gelöst und unter Argongegenstrom in einen 100 mL Edelstahlautoklav gefüllt. Anschließend wurden ~5 bar CO/H₂ (1:1) angelegt und innerhalb von 10 min auf 100 °C erwärmt. Bei dieser Temperatur wurde der Druck auf 10 bar erhöht und 10 h gerührt. Der Autoklav wurde auf Raumtemperatur abgekühlt, auf Atmosphärendruck entspannt und ein Aliquot des erhaltenen Reaktionsaustrags mittels Gaschromatographie analysiert. GC-Methode: Optima1 Säule (30 m, d = 320 µm, F_{d} = 0.5 µm), Temperaturprogramm: 50°C für 2 min, mit 5°C/min auf 90°C und für 2 min isotherm, mit 20°C/min auf 250°C, Helium als Trägergas. t_{R}(2,4,4-Trimethylpent-1-en) = 5.27 min, t_{R}(2,4,4-Trimethylpent-2-en) = 5.77 min, t_{R}(3,5,5-Trimethylhexanal) = 13.76 min).

Es wurde folgendes Ergebnis erzielt:

| | |
|---|---|
| Umsatz von Diisobuten: | >98% |
| Selektivität zu 3,5,5-Trimethylhexanal: | 97.0% |
| Selektivität zu 2,4,4-Trimethylpentan: | 3.0% |

### Beispiel 2:

Unter Argon wurden Rh(CO)₂(acac) (5.1 mg, 0.02 mmol) und Tris(2,4-di-tert-butylphenyl)phosphit (640 mg, 0.99 mmol) in Toluol (2.5 mL) gelöst und in einen 100 mL Edelstahlautoklav gefüllt. Anschließend wurden 10 bar CO/H₂ (1:1) angelegt, innerhalb von 10 min auf 100 °C erwärmt und 1 h gerührt. Bei dieser Temperatur wurde Diisobuten (20.0 g, 178 mmol, 2,4,4-Tri-methylpent-1-en / 2,4,4-Trimethylpent-2-en: 80:20 bis 75:25) im Gegenstrom hinzugefügt und 6 h gerührt. Der Autoklav wurde auf Raumtemperatur abgekühlt, auf Atmosphärendruck entspannt und ein Aliquot des erhaltenen Reaktionsaustrags mittels Gaschromatographie analysiert. GC-Methode: Optima1 Säule (30 m, d = 320 µm, Fd = 0.5 µm), Temperaturprogramm: 50 °C für 2 min, mit 5 °C/min auf 90 °C und für 2 min isotherm, mit 20 °C/min auf 250 °C, Helium als Trägergas. t_{R}(2,4,4-Trimethylpent-1-en) = 5.27 min, t_{R}(2,4,4-Trimethylpent-2-en) = 5.77 min, t_{R}(3,5,5-Trimethylhexanal) = 13.76 min).

Es wurde folgendes Ergebnis erzielt:

| | |
|---|---|
| Umsatz von Diisobuten: | >95% |

| | |
|---|---|
| Selektivität zu 3,5,5-Trimethylhexanal: | 97.0% |
| Selektivität zu 2,4,4-Trimethylpentan: | 3.0% |

### Beispiel 3:

Unter Argon wurden Rh(CO)₂(acac) (5.1 mg, 0.02 mmol) Tris(2,4-di-tert-butylphenyl)phosphit (640 mg, 0.99 mmol) in 2,4,4-Trimethylpent-2-en (20.0 g, 178 mmol) gelöst und unter Argongegenstrom in einen 100 mL Edelstahlautoklav gefüllt. Anschließend wurden ~5 bar CO/H₂ (1:1) angelegt und innerhalb von 10 min auf 100 °C erwärmt. Bei dieser Temperatur wurde der Druck auf 10 bar erhöht und 10 h gerührt. Der Autoklav wurde auf Raumtemperatur abgekühlt, auf Atmosphärendruck entspannt und ein Aliquot des erhaltenen Reaktionsaustrags mittels Gaschromatographie analysiert. GC-Methode: Optima1 Säule (30 m, d = 320 µm, F_{d} = 0.5 µm), Temperaturprogramm: 50 °C für 2 min, mit 5 °C/min auf 90 °C und für 2 min isotherm, mit 20 °C/min auf 250 °C, Helium als Trägergas. t_{R}(2,4,4-Trimethylpent-1-en) = 5.27 min, t_{R}(2,4,4-Trimethyl-pent-2-en) = 5.77 min, t_{R}(3,5,5-Trimethylhexanal) = 13.76 min).

Es wurde folgendes Ergebnis erzielt:

| | |
|---|---|
| Umsatz von Diisobuten: | >85% |
| Selektivität zu 3,5,5-Trimethylhexanal: | 96.0% |
| Selektivität zu 2,4,4-Trimethylpentan: | 4.0% |

### Beispiel 4:

Unter Argon wurden eine Lösung von Tris(2,4-di-tert-butylphenyl)phosphit L1 in 3,5,5-Trimethylhexanal (10 gew%) unter Rückfluss gerührt. Nach 1 d, 5 d, 8 d, 12 d, 21 d und 27 d wurden Proben mittels ³¹P-NMR-Spektroskopie analysiert (Tris(2,4-di-tert-butylphenyl)phosphit: δ = 129.9 ppm; Tris(2,4-di-tert-butylphenyl)phosphat δ = -20.0 ppm).

Es wurde folgendes Ergebnis erzielt:

| Reaktionszeit | 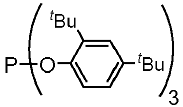 ³¹P-NMR (129.9 ppm) | 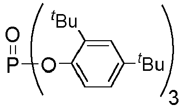 ³¹P-NMR(-20.0 ppm) |
|---|---|---|
| 1 d | >99% | <1% |
| 5 d | >99% | <1% |
| 8 d | 99% | 1% |
| 12 d | 98% | 2% |
| 21 d | 97% | 3% |
| 27 d | 97% | 3% |

Wie das Beispiel zeigt, weist der Ligand eine hohe Stabilität im Reaktionsprodukt auf. Durch die hohe Stabilität des Liganden ist eine lange Standzeit für das Verfahren gewährleistet. Weiterhin birgt die hohe Stabilität der Liganden den Vorteil, dass die Nebenproduktbildung reduziert ist. Zudem können die Liganden und/oder der Katalysatormetall-Liganden-Komplex im Fall einer kontinuierlichen Verfahrensführung aufgrund ihrer hohen Stabilität in das Verfahren zurückgeführt werden.

Die Beispiele dienen der Veranschaulichung der vorliegenden Erfindung. Die Beispiele schränken die vorliegende Erfindung nicht ein.

## Patentansprüche

1. Hydroformylierungsverfahren zur Herstellung von 3,5,5-Trimethylhexanal umfassend die Umsetzung von 2,4,4-Trimethylpent-2-en mit H₂ und CO in einer Reaktionszone in Gegenwart von einem oder mehreren freien Organophosphit-Liganden der allgemeinen Formel (I) 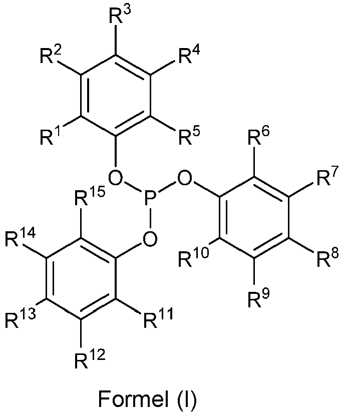 wobei,
R¹, R², R³, R⁴ und R⁵ unabhängig voneinander H, C₁- bis C₉-Alkyl oder C₁- bis C₉-Alkoxyl sind und R¹, R², R³, R⁴ und R⁵ nicht gleichzeitig H sind, und
R⁶, R⁷, R⁸, R⁹ und R¹⁰ unabhängig voneinander H, C₁- bis C₉-Alkyl oder C₁- bis C₉-Alkoxyl sind und R⁶, R⁷, R⁸, R⁹ und R¹⁰ nicht gleichzeitig H sind, und
R¹¹, R¹², R¹³, R¹⁴ und R¹⁵ unabhängig voneinander H, C₁- bis C₉-Alkyl oder C₁- bis C₉-Al-koxyl sind und R¹¹, R¹², R¹³, R¹⁴ und R¹⁵ nicht gleichzeitig H sind,
und eines mit einem oder mehreren Organophosphit-Liganden der allgemeinen Formel (I) komplexierten, homogenen Rhodium-Katalysators bei einem Druck von 1 bis 100 bar abs und einer Temperatur von 50 bis 200 °C.

2. Verfahren nach Anspruch 1, wobei eine Mischung enthaltend 2,4,4-Trimethyl-pent-1en und 2,4,4-Trimethylpent-2-en zu 3,5,5-Trimethylhexanal umgesetzt wird.

3. Verfahren nach Anspruch 2, wobei das Mengenverhältnis von 2,4,4-Trimethylpent-1-en zu 2,4,4-Trimethylpent-2-en in der eingesetzten Mischung im Bereich von 99 : 1 bis 1 : 99 liegt.

4. Verfahren nach einem der Ansprüche 1 bis 3, wobei die Gesamtmenge an 2,4,4-Trime-thylpent-1-en und 2,4,4-Trimethylpent-2-en an der Gesamtmenge an Olefin im Feed zur Reaktionszone 10 bis 100 % beträgt.

5. Verfahren nach einem der Ansprüche 1 bis 4, wobei die Hydroformylierung bei einer Temperatur von 80 bis 150 °C durchgeführt wird.

6. Verfahren nach einem der Ansprüche 1 bis 5, wobei die Hydroformylierung bei einem Druck von 5 bis 80 bar abs durchgeführt wird.

7. Verfahren nach einem der Ansprüche 1 bis 6, wobei das Molverhältnis der Gesamtmenge an Organophosphit-Liganden der allgemeinen Formel (I) in der Reaktionszone zur Gesamtmenge an Rhodium in der Reaktionszone im Bereich von 1 : 1 bis 100 : 1 liegt.

8. Verfahren nach einem der Ansprüche 1 bis 7, wobei die Rhodiumkonzentration in der Reaktionszone im Bereich von 20 bis 250 Gew. ppm bezogen auf das Gesamtgewicht der flüssigen Phase in der flüssigen Phase in der Reaktionszone liegt.

9. Verfahren nach einem der Ansprüche 1 bis 8, wobei es sich bei dem einen oder mehreren Organophosphit-Liganden der allgemeinen Formel (I) um eine oder mehrere der nachfolgenden Verbindungen handelt:
| Organophosphit-Ligand der allgemeinen Formel (I) | R¹, R⁶, R¹¹ | R³, R⁸, R¹³ | R², R⁴, R⁵, R⁷, R⁹, R¹⁰, R¹², R¹⁴, R¹⁵ |
|---|---|---|---|
| I) | Methyl | Methyl | H |
| II) | Methyl | H | H |
| III) | Ethyl | Ethyl | H |
| IV | Ethyl | H | H |
| V) | n-Propyl | n-Propyl | H |
| VI) | n-Propyl | H | H |
| VII) | iso-Propyl | iso-Propyl | H |
| VIII) | iso-Propyl | H | H |
| IX) | n-Butyl | n-Butyl | H |
| X) | n-Butyl | H | H |
| XI) | sec-Butyl | sec-Butyl | H |
| XII) | sec-Butyl | H | H |
| XIII) | iso-Butyl | iso-Butyl | H |
| XIV) | iso-Butyl | H | H |
| XV) | tert-Butyl | tert-Butyl | H |
| XVI) | tert-Butyl | H | H |
| XVII) | Methoxy | Methoxy | H |
| XVIII) | Methoxy | H | H |
| XIX) | Ethoxy | Ethoxy | H |
| XX) | n-Propoxy | n-Propoxy | H |
| XXI) | n-Propoxy | H | H |
| XXII) | iso-Propoxy | iso-Propoxy | H |
| XXIII) | iso-Propoxy | H | H |
| XXIV) | n-Butoxy | n-Butoxy | H |
| XXV) | n-Butoxy | H | H |
| XXVI) | sec-Butoxy | sec-Butoxy | H |
| XXVII) | sec-Butoxy | H | H |
| XXVIII) | iso-Butoxy | iso-Butoxy | H |
| XXIX) | iso-Butoxy | H | H |
| XXX) | tert-Butoxy | tert-Butoxy | H |
| XXXI) | tert-Butoxy | H | H |
| XXXII) | n-Nonyl | H | H |

10. Verfahren nach einem der Ansprüche 1 bis 9, wobei das Molverhältnis von H₂ zu CO die der Reaktionszone zugeführt werden 2 : 1 bis 1 : 2 beträgt.

11. Verfahren nach einem der Ansprüche 1 bis 10, wobei der Rhodiumkatalysator zumindest teilweise in der Reaktionszone durch Umsetzung eines Rhodium-Präkursors mit einem oder mehreren Organophosphit-Liganden der allgemeinen Formel (I), wie in Anspruch 1 oder 9 definiert, CO und H₂ gebildet wird.

12. Verfahren nach Anspruch 11, wobei es sich bei dem Rhodium-Präkursor um Rhodiumcarbonyle, Rhodium(I)-Salze, Rhodium(II)-Salze, Rhodium(III)-Salze oder Mischungen aus zwei oder mehr der zuvor genannten Präkursoren handelt.

13. Verfahren nach einem der Ansprüche 1 bis 10, wobei der Rhodiumkatalysator zumindest teilweise präformiert eingesetzt wird.

14. Verfahren nach einem der Ansprüche 1 bis 13, wobei das Verfahren kontinuierlich ausgeführt wird.

## Claims

1. A hydroformylation process for preparing 3,5,5-trimethylhexanal comprising reacting 2,4,4-trimethylpent-2-ene with H₂ and CO in a reaction zone in the presence of one or more free organophosphite ligands of the general formula (I) 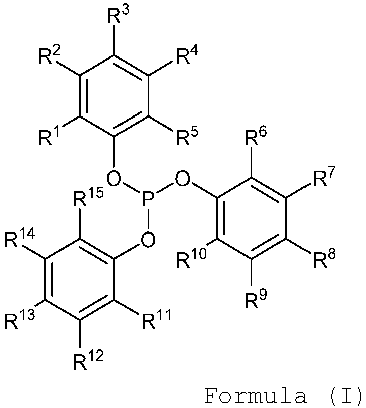 wherein,
R¹, R², R³, R⁴ and R⁵ are each independently H, C₁- to C₉-alkyl or C₁- to C₉-alkoxy and R¹, R², R³, R⁴ and R⁵ are not H at the same time, and
R⁶, R⁷, R⁸, R⁹ and R¹⁰ are each independently H, C₁- to C₉-alkyl or C₁- to C₉-alkoxy and R⁶, R⁷, R⁸, R⁹ and R¹⁰ are not H at the same time, and
R¹¹, R¹², R¹³, R¹⁴ and R¹⁵ are each independently H, C₁-to C₉-alkyl or C₁- to C₉-alkoxy and R¹¹, R¹², R¹³, R¹⁴ and R¹⁵ are not H at the same time,
and a homogeneous rhodium catalyst complexed with one or more organophosphite ligands of the general formula (I) at a pressure of 1 to 100 bar abs and a temperature of from 50 to 200°C.

2. The process according to claim 1, wherein a mixture comprising 2,4,4-_{trimethylpent}-1-ene and 2, 4, 4-trimethylpent-2-ene is converted to 3,5,5-trimethylhexanal.

3. The process according to claim 2, wherein the ratio of 2, 4, 4-trimethylpent-1-ene to 2, 4, 4-trimethylpent-2-ene in the mixture used is in the range from 99 : 1 to 1 : 99.

4. The process according to any of claims 1 to 3, wherein the total amount of 2, 4, 4-trimethylpent-1-ene and 2,4,4-trimethylpent-2-ene of the total amount of olefin in the feed to the reaction zone is from 10 to 100%.

5. The process according to any of claims 1 to 4, wherein the hydroformylation is carried out at a temperature of 80 to 150°C.

6. The process according to any of claims 1 to 5, wherein the hydroformylation is carried out at a pressure of 5 to 80 bar abs.

7. The process according to any of claims 1 to 6, wherein the molar ratio of the total amount of organophosphite ligands of the general formula (I) in the reaction zone to the total amount of rhodium in the reaction zone is in the range from 1 : 1 to 100 : 1.

8. The process according to any of claims 1 to 7, wherein the rhodium concentration in the reaction zone is in the range of 20 to 250 ppmw based on the total weight of the liquid phase in the liquid phase in the reaction zone.

9. The process according to any of claims 1 to 8, wherein the one or more organophosphite ligands of the general formula (I) are one or more of the following compounds:
| organophosphite ligand of the general formula (I) | R¹, R⁶, R¹¹ | R³, R⁸, R¹³ | R², R⁴, R⁵, R⁷, R⁹, R¹⁰, R¹², R¹⁴, R¹⁵ |
|---|---|---|---|
| I) | Methyl | Methyl | H |
| II) | Methyl | H | H |
| III) | Ethyl | Ethyl | H |
| IV) | Ethyl | H | H |
| V) | n-Propyl | n-Propyl | H |
| VI) | n-Propyl | H | H |
| VII) | Isopropyl | Isopropyl | H |
| VIII) | Isopropyl | H | H |
| IX) | n-Butyl | n-Butyl | H |
| X) | n-Butyl | H | H |
| XI) | sec-Butyl | sec-Butyl | H |
| XII) | sec-Butyl | H | H |
| XIII) | Isobutyl | Isobutyl | H |
| XIV) | Isobutyl | H | H |
| XV) | tert-Butyl | tert-Butyl | H |
| XVI) | tert-Butyl | H | H |
| XVII) | Methoxy | Methoxy | H |
| XVIII) | Methoxy | H | H |
| XIX) | Ethoxy | Ethoxy | H |
| XX) | n-Propoxy | n-Propoxy | H |
| XXI) | n-Propoxy | H | H |
| XXII) | Isopropoxy | Isopropoxy | H |
| XXIII) | Isopropoxy | H | H |
| XXIV) | n-Butoxy | n-Butoxy | H |
| XXV) | n-Butoxy | H | H |
| XXVI) | sec-Butoxy | sec-Butoxy | H |
| XXVII) | sec-Butoxy | H | H |
| XXVIII) | Isobutoxy | Isobutoxy | H |
| XXIX) | Isobutoxy | H | H |
| XXX) | tert-Butoxy | tert-Butoxy | H |
| XXXI) | tert-Butoxy | H | H |
| XXXII) | n-Nonyl | H | H |

10. The process according to any of claims 1 to 9, wherein the molar ratio of H₂ to CO fed to the reaction zone is from 2 : 1 to 1 : 2.

11. The process according to any of claims 1 to 10, wherein the rhodium catalyst is at least partially formed in the reaction zone by reacting a rhodium precursor with one or more organophosphite ligands of the general formula (I), as defined in claim 1 or 9, CO and H₂.

12. The process according to claim 11, wherein the rhodium precursor is rhodium carbonyls, rhodium(I) salts, rhodium(II) salts, rhodium(III) salts or mixtures of two or more of the aforementioned precursors.

13. The process according to any of claims 1 to 10, wherein the rhodium catalyst is used at least partially preformed.

14. The process according to any of claims 1 to 13, wherein the method is carried out continuously.

## Revendications

1. Procédé d'hydroformylation pour la préparation de 3,5,5-triméthylhexanal comprenant la transformation de 2,4,4-triméthylpent-2-ène avec H₂ et CO dans une zone de réaction en présence d'un ou de plusieurs ligands organophosphite libres de formule générale (I) 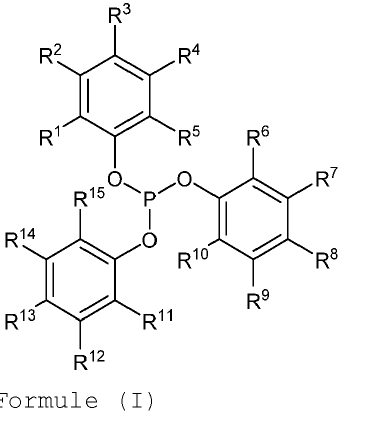 dans laquelle
R¹, R², R³, R⁴ et R⁵ représentent, indépendamment les uns des autres, H, C₁-C₉-alkyle ou C₁-C₉-alcoxyle et R¹, R², R³, R⁴ et R⁵ ne représentent pas simultanément H et
R⁶, R⁷, R⁸, R⁹ et R¹⁰ représentent, indépendamment les uns des autres, H, C₁-C₉-alkyle ou C₁-C₉-alcoxyle et R⁶, R⁷, R⁸, R⁹ et R¹⁰ ne représentent pas simultanément H et
R¹¹, R¹², R¹³, R¹⁴ et R¹⁵ représentent, indépendamment les uns des autres, H, C₁-C₉-alkyle ou C₁-C₉-alcoxyle et R¹¹, R¹², R¹³, R¹⁴ et R¹⁵ ne représentent pas simultanément H
et d'un catalyseur à base de rhodium homogène, complexé par un ou plusieurs ligands organophosphite de formule générale (I) à une pression de 1 à 100 bars abs et à une température de 50 à 200°C.

2. Procédé selon la revendication 1, un mélange contenant du 2,4,4-triméthylpent-1-ène et du 2,4,4-triméthylpent-2-ène étant transformé en 3,5,5-triméthylhexanal.

3. Procédé selon la revendication 2, le rapport des quantités de 2,4,4-triméthylpent-1-ène à 2,4,4-triméthylpent-2-ène dans le mélange utilisé étant situé dans la plage de 99:1 à 1:99.

4. Procédé selon l'une des revendications 1 à 3, la quantité totale de 2,4,4-triméthylpent-1-ène et de 2,4,4-triméthylpent-2-ène par rapport à la quantité totale d'oléfine dans l'alimentation dans la zone de réaction valant 10 à 100%.

5. Procédé selon l'une des revendications 1 à 4, l'hydroformylation étant réalisée à une température de 80 à 150°C.

6. Procédé selon l'une des revendications 1 à 5, l'hydroformylation étant réalisée à une pression de 5 à 80 bars abs.

7. Procédé selon l'une des revendications 1 à 6, le rapport molaire de la quantité totale de ligands organophosphite de formule générale (I) dans la zone de réaction à la quantité totale de rhodium dans la zone de réaction étant situé dans la plage de 1:1 à 100:1.

8. Procédé selon l'une des revendications 1 à 7, la concentration en rhodium dans la zone de réaction étant située dans la plage de 20 à 250 ppm en poids par rapport au poids total de la phase liquide dans la phase liquide dans la zone de réaction.

9. Procédé selon l'une quelconque des revendications 1 à 8, où il s'agit, pour ledit un ou lesdits plusieurs ligands organophosphite de formule générale (I) d'un ou de plusieurs des composés suivants :
| Ligand organophosphite de formule générale (I) | R¹, R⁶, R¹¹ | R³, R⁸, R¹³ | R², R⁴, R⁵, R⁷, R⁹, R¹⁰, R¹², R¹⁴, R¹⁵ |
|---|---|---|---|
| I) | méthyle | méthyle | H |
| II) | méthyle | H | H |
| III) | éthyle | éthyle | H |
| IV) | éthyle | H | H |
| V) | n-propyle | n-propyle | H |
| VI) | n-propyle | H | H |
| VII) | iso-propyle | iso-propyle | H |
| VIII) | iso-propyle | H | H |
| IX) | n-butyle | n-butyle | H |
| X) | n-butyle | H | H |
| XI) | sec-butyle | sec-butyle | H |
| XII) | sec-butyle | H | H |
| XIII) | iso-butyle | iso-butyle | H |
| XIV) | iso-butyle | H | H |
| XV) | tert-butyle | tert-butyle | H |
| XVI) | tert-butyle | H | H |
| XVII) | méthoxy | méthoxy | H |
| XVIII) | méthoxy | H | H |
| XIX) | éthoxy | éthoxy | H |
| XX) | n-propoxy | n-propoxy | H |
| XXI) | n-propoxy | H | H |
| XXII) | iso-propoxy | iso-propoxy | H |
| XXIII) | iso-propoxy | H | H |
| XXIV) | n-butoxy | n-butoxy | H |
| XXV) | n-butoxy | H | H |
| XXVI) | sec-butoxy | sec-butoxy | H |
| XXVII) | sec-butoxy | H | H |
| XXVIII) | iso-butoxy | iso-butoxy | H |
| XXIX) | iso-butoxy | H | H |
| XXX) | tert-butoxy | tert-butoxy | H |
| XXXI) | tert-butoxy | H | H |
| XXXII) | n-nonyle | H | H |

10. Procédé selon l'une des revendications 1 à 9, le rapport molaire de H₂ à CO qui sont introduits dans la zone de réaction étant de 2:1 à 1:2.

11. Procédé selon l'une des revendications 1 à 10, le catalyseur de rhodium étant formé au moins en partie dans la zone de réaction par transformation d'un précurseur de rhodium avec un ou plusieurs ligands organophosphite de formule générale (I), telle que définie dans la revendication 1 ou 9, CO et H₂.

12. Procédé selon la revendication 11, dans lequel il s'agit, pour le précurseur de rhodium, de rhodiumcarbonyle, de sels de rhodium (I), de sels de rhodium (II), de sels de rhodium (III) ou de mélanges de deux, ou plus, des précurseurs susmentionnés.

13. Procédé selon l'une des revendications 1 à 10, le catalyseur de rhodium étant utilisé sous une forme au moins partiellement préformée.

14. Procédé selon l'une des revendications 1 à 13, le procédé étant mis en œuvre en continu.
